# EUROPEAN PATENT APPLICATION

(11) **EP 1 655 604 A1**
(43) Date of publication of application: **10.05.2006**
(21) Application number: 04746355.9
(22) Date of filing: 24.06.2004
(51) Int. Cl.: G01N 33/52

(54) **ANALYTICAL TEST PIECE AND PROCESS FOR PRODUCING THE SAME**

(30) Priority: 24.06.2003 JP 2003180337; 24.06.2003 JP 2003180338; 03.10.2003 JP 2003345868; 16.04.2004 JP 2004122119
(71) Applicant: NGK INSULATORS, LTD., Nagoya-shi, Aichi 467-8530 (JP)
(72) Inventor: OHNISHI, Takao, c/o NGK Insulators, Ltd., Nagoya-shi Aichi 467-8530 (JP); HIROTA, Toshikazu, c/o NGK Insulators, Ltd., Nagoya-shi Aichi 467-8530 (JP)
(74) Representative: Paget, Hugh Charles Edward
(86) International application number: PCT/JP2004/008886
(87) International publication number: WO 2004/113916

(57) **Abstract**

Analytical test piece (10) comprising a support (1) and, disposed on the surface (11) and/or in the interior of the support (1), reagent spots (2), the analytical test piece (10) adapted, when an analyte-containing sample (3) is introduced onto the surface (11) of the support (1), to cause the analyte-containing sample (3) to be brought into contact with and react with the reagent spots (2) disposed on the surface (11) and/or in the interior of the support (1) and produce a detectable substance (a signal substance) or exhibit a detectable property (or signal property), wherein the reagent spots (2) are composed of two or more types of reagent spots (21, 22) constituted of any of two or more types of solutions, each of which exhibits a given function upon mixing with the other, the sample (3) introduced in the surface (11) of the support (1) being brought into contact with the two or more types of reagent spots (21, 22) so as to effect not only mutual mixing of the multiple types of reagent spots (21, 22) but also reacting with the two or more types of reagent spots (21, 22) having been mixed together, thereby forming a signal substance or exhibiting a signal property. This analytical test piece excels in analytical reliability, analytical sensitivity (analytical precision), and storage stability.

## Description

### TECHNICAL FIELD

The present invention relates to an analytical test piece comprising reagent spots which come in contact with and react with an analyte-containing sample introduced therein and produce a detectable substance (a signal substance) or exhibit a detectable property (a signal property) and to a process for efficiently producing the analytical test piece. More particularly, the present invention relates to an analytical test piece useful as an inspection chip for inspecting and analyzing samples containing an analyte (e.g. a body fluid, particularly urine, blood, etc. of humans and animals) and excelling in analysis reliability, analytical sensitivity (analytical precision), and storage stability, and to a process for efficiently producing the analytical test piece.

### BACKGROUND ART

As an example of the method for preparing an analytical test piece for inspecting and analyzing samples containing an analyte (e.g. a body fluid, particularly urine, blood, etc. of humans and animals), Patent Document 1 discloses a porous film and a method for preparing the same, wherein the porous film has a test part comprising a highly absorptive porous structure (such as a porous layer, porous membrane, etc.) which can uniformly absorb a sample liquid while preventing the liquid from communicating with the liquid in the next adjoining test part. Patent Document 2 discloses an analytical test piece provided with one or more test parts having a detector for detecting a detectable substance, in which the detector comprises a stratified inorganic compound (synthetic smectite, etc.) and a method for preparing the analytical test piece.

[Patent Document 1] Japanese Patent Application Laid-open No. H02-6541 [Patent Document 2] Japanese Patent Application Laid-open No. H09-184837

However, the porous film, the analytical test piece, and the methods for preparation disclosed in the Patent Documents 1 and 2 are not necessarily satisfactory. Since the analysis requires causing two or more samples to be dipped in a single buffer, the sample on the resulting analytical test piece deteriorates in a short time, lacks stability, exhibits only low reactivity, and cannot be analyzed at high sensitivity and high precision.

The present invention has been achieved in view of the above situation and has an object of providing an analytical test piece useful as an inspection chip for inspecting and analyzing samples containing an analyte (e.g. a body fluid, particularly urine, blood, etc. of humans and animals) and excelling in analysis reliability, analytical sensitivity (analytical precision), and storage stability, and a process for efficiently producing the analytical test piece.

### DISCLOSURE OF THE INVENTION

Specifically, the present invention provides the following analytical test piece and the method for preparing the same.

(1) An analytical test piece comprising a support 1 and reagent spots aligned on the surface of the support or on the surface and the inside of the support in a specified spot pattern (a spot pitch), wherein an analyte-containing sample introduced onto the surface 11 of the support 1 comes in contact with and reacts with the reagent spots aligned on the surface of the support or on the surface and inside of the support, thereby producing a detectable substance (a signal substance) or exhibiting a detectable property (a signal property), the reagent spots being two or more types of reagent spots formed of any of two or more types of solutions which exhibit a given function when mixed with the other types of solutions, and the sample introduced to the surface of the support coming in contact with the two or more types of reagent spots, causing the reagent spots to mix together, and reacting with the mixed reagent spots, thereby forming the signal substance or exhibiting the signal property.

(2) The analytical test piece according to (1) above, wherein the support is a porous body.

(3) The analytical test piece according to the above (1) or (2), wherein the analyte is a human body fluid or animal body fluid.

(4) The analytical test piece according to any of (1) to (3) above, wherein at least one of the solutions forming the reagent spots comprises a coloring substance.

(5) The analytical test piece according to any of (1) to (4) above, wherein at least one of the solutions forming the reagent spots comprises a fluorescent substance.

(6) The analytical test piece according to any of (1) to (5) above, wherein at least one of the solutions forming the reagent spots comprises a water-soluble polymer.

(7) The analytical test piece according to any of (1) to (6) above, wherein water-soluble shields are disposed in spaces in which reagent spots are not aligned (between reagent spots) on the surface of the support or on the surface and inside of the support.

(8) The analytical test piece according to any of (1) to (7) above, wherein the reagent spots aligned in at least one area on the surface of the support or on the surface and inside of the support are types different from the reagent spots aligned in the other areas.

(9) The analytical test piece according to any of (1) to (8) above, wherein the concentration of reagent spots aligned in at least one area on the surface of the support or on the surface and inside of the support is different from the concentration of the reagent spots aligned in the other areas.

(10) The analytical test piece according to any of (1) to (9) above, wherein when the reagent spots are aligned not only on the surface, but also inside the support, the reagent spots are aligned so that those on the surface of the support exhibit the highest reactivity with the samples.

(11) The analytical test piece according to any of (1) to (9) above, wherein when the reagent spots are aligned not only on the surface, but also inside the support, the reagent spots are aligned so that those on the surface of the support have the highest concentration.

(12) The analytical test piece according to any of (1) to (11) above, wherein a surfactant is disposed in spaces in which reagent spots are not aligned (between reagent spots) on the surface of the support or on the surface and inside of the support.

(13) The analytical test piece according to any of (1) to (12) above, wherein a foaming agent is disposed in spaces in which reagent spots are not aligned (between reagent spots) on the surface of the support or on the surface and inside of the support.

(14) The analytical test piece according to any of (1) to (13) above, further provided with a supporting body for supporting the above support on the opposite surface (back) to the surface of the above support.

(15) The analytical test piece according to any of (1) to (14) above, wherein the cross-section of the reagent spots cut along a specified plane parallel to the surface of the support is oval or elliptical, or has the shape of a race track.

(16) The analytical test piece according to any of (1) to (15) above, wherein the distance between spot centers (spot pitch) of the same reagent (L2) is greater than the distance between spot centers (spot pitch) of the two different reagents (L1).

(17) The analytical test piece according to any of (1) to (16) above, wherein the diameter of the reagent spots is 0.5 mm or less and the distance (spot pitch) between the center of the different reagent spots (L1) is 0.6 mm or less.

(18) The analytical test piece according to any of (1) to (17) above, wherein an alignment ratio (A) of the reagent spots on the surface of the support or on the surface and inside of the support, expressed by the formula (A) = (S1/S2), satisfies an inequality of 0.01 < (A) < 0.814, wherein S 1 indicates the total cross-sectional areas of the reagent spots in a specified plane parallel to the surface of the support and S2 is the area of the surface of the support.

(19) The analytical test piece according to any of (1) to (18) above, wherein a spot pitch, which is the aligning pattern of the reagent spots on the surface of the support or on the surface and inside of the support, in a certain area differs from the spot pitch in other areas.

(20) The analytical test piece according to any of (1) to (19) above, further provided with confirmation reagent spots outside the area in which the reagent spots are aligned according to the specified pattern (spot pitch), for previously confirming proper alignment of the spots on the surface of the support or on the surface and in the inside of the support.

(21) The analytical test piece according to any of (1) to (20) above, wherein the amount of the solution (spot amount) in one reagent spot in at least one area on the surface of the support or on the surface and inside of the support differs from the spot amount in other areas.

(22) A method for preparing an analytical test piece comprising reagent spots formed by aligning prescribed solutions on the surface of a support or on the surface and inside of the support in a prescribed spot alignment pattern (a spot pitch), with which an analyte-containing sample introduced onto the surface of the support is brought into contact and reacts, thereby producing a detectable substance (a signal substance) or exhibiting a detectable property (a signal property), the method comprising providing two or more types of solutions, which are being mixed together when brought into contact with the above sample to be introduced and each of which exhibits a given function when mixed together, as the above prescribed solutions, and injecting the solutions onto the surface of the support or the surface and the inside of the support using an inkjet method to form two or more reagent spots, each consisting of any of the two or more types of solutions, so that the above sample introduced onto the surface of the support comes in contact with the reagent spots formed on the surface of the support or on the surface and inside of the support, causes the reagent spots to mix, and reacts with the mixed reagent spots to produce a signal substance or exhibit a signal property.

(23) The method according to (22) above, wherein a liquid drop injector comprising a fluid channel substrate in which a fluid channel is formed, an actuator installed in the fluid channel substrate having a function of changing the volume of a cavity as a pressurizing chamber, a nozzle substrate attached to the bottom of the fluid channel substrate with nozzles formed therein, and a liquid receiver installed on the rear top of the fluid channel substrate is used in the inkjet method.

(24) The method according to (22) or (23) above, wherein a porous material is used as the support.

(25) The method according to any of (22) to (24) above, wherein the analyte is a human body fluid or animal body fluid.

(26) The method according to any of (22) to (25) above, wherein at least one of the solutions forming the reagent spots comprises a coloring substance.

(27) The method according to any of (22) to (26) above, wherein at least one of the solutions forming the reagent spots comprises a fluorescent substance.

(28) The method according to any of (22) to (27) above, wherein at least one of the solutions forming the reagent spots comprises a water-soluble polymer.

(29) The method according to any of (22) to (28) above, wherein water-soluble shields are disposed in spaces in which reagent spots are not aligned (between reagent spots) on the surface of the support or on the surface and inside of the support.

(30) The method according to any of (22) to (29) above, wherein the cross-section of the reagent spots cut along a specified plane parallel to the surface of the support is oval or elliptical, or has the shape of a race track.

(31) The method according to any of (22) to (30) above, wherein the reagent spots are aligned so that the distance between spot centers (spot pitch) of the same reagent (L2) may be greater than the distance between spot centers (spot pitch) of the two different reagents (L1).

(32) The method according to any of (22) to (31) above, wherein the diameter of the reagent spots is 0.5 mm or less and the distance (spot pitch) between the center of the different reagent spots (L1) is 0.6 mm or less.

(33) The method according to any of (22) to (32) above, wherein the reagent spots are aligned so that an alignment ratio (A) on the surface of the support or on the surface and inside of the support, expressed by the formula (A) = (S1/S2), satisfies an inequality of 0.01 < (A) < 0.814, wherein S 1 indicates the total cross-sectional areas of the reagent spots in a specified plane parallel to the surface of the support and S2 is the area of the surface of the support.

(34) The method according to any of (22) to (33) above, wherein the reagent spots are aligned so that the spot pitch, which is the aligning pattern of the reagent spots on the surface of the support or on the surface and inside of the support, in a certain area may differ from the spot pitch in other areas.

(35) The method according to any of (22) to (34) above, wherein confirmation reagent spots are further provided outside the area in which the reagent spots are aligned according to the specified pattern (spot pitch), for previously confirming proper alignment of the spots on the surface of the support or on the surface and in the inside of the support.

(36) The method according to (35) above, wherein each of the confirmation reagent spots contains one of the above-mentioned solutions.

(37) The method according to any of (22) to (36) above, wherein the reagent spots are aligned so that the reagent spots aligned in at least one area on the surface of the support or on the surface and inside of the support are types different from the reagent spots aligned in the other areas.

(38) The method according to any of (22) to (37) above, wherein the reagent spots are aligned so that the reagent spots aligned in at least one area on the surface of the support or on the surface and inside of the support have a concentration different from the reagent spots aligned in the other areas.

(39) The method according to any of (22) to (38) above, wherein when the reagent spots are aligned not only on the surface, but also inside the support, the reagent spots are aligned so that those on the surface of the support exhibit the highest reactivity with the samples.

(40) The method according to any of (22) to (39) above, wherein when the reagent spots are aligned not only on the surface, but also inside the support, the reagent spots are aligned so that those on the surface of the support exhibit the highest concentration.

(41) The method according to any of (22) to (40) above, wherein a surfactant is disposed in spaces in which reagent spots are not aligned (between reagent spots) on the surface of the support or on the surface and inside of the support.

(42) The method according to any of (22) to (41) above, wherein a foaming agent is disposed in spaces in which reagent spots are not aligned (between reagent spots) on the surface of the support or on the surface and inside of the support.

(43) The analytical test piece according to any of (22) to (42) above, wherein a supporting body for supporting the above support is provided on the opposite surface (back) to the surface of the above support.

(44) The method according to any of (22) to (43) above, wherein the reagent spots are formed by injecting liquid drops of the above two or more solutions using the inkjet method so that some solutions may be injected in a direction other than the vertical direction to the surface of the support.

(45) The method according to any of (22) to (44) above, wherein many very small independent droplets of the above solutions are produced.

(46) The method according to any of (22) to (45) above, wherein the amount of liquid drops forming one reagent spot (spot amount) is 0.1 µl or less.

(47) The method according to any of (22) to (46) above, wherein the amount of the solution (spot amount) in one reagent spot in at least one area on the surface of the support or on the surface and inside of the support differs from the spot amount in other areas.

(48) The method according to any of (22) to (47) above, wherein the reagent spots are formed by injecting droplets of the solution two or more times per one reagent spot.

(49) The method according to any of (22) to (48) above, wherein the reagent spots are produced by injecting the liquid drops of the above solutions while maintaining the temperature of the support at 40°C or less.

(50) The method according to any of (43) to (49) above, wherein the reagent spots are produced by injecting the liquid drops of the above solutions while the back of the support 1 is separated from the supporting body 4.

As described above, an analytical test piece useful as an inspection chip for inspecting and analyzing samples containing an analyte (e.g. a body fluid, particularly urine, blood, etc. of humans and animals) and excelling in analysis reliability, analytical sensitivity (analytical precision), and storage stability, as well as a method for efficiently producing the analytical test piece are provided by the present invention.

### BRIEF DESCRIPTION OF THE DRAWING

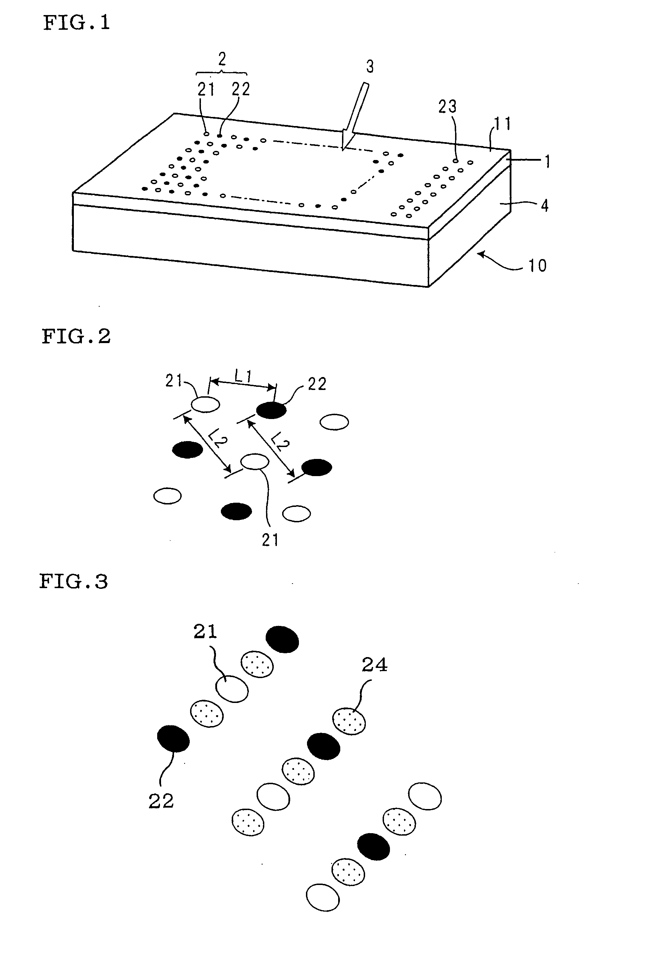

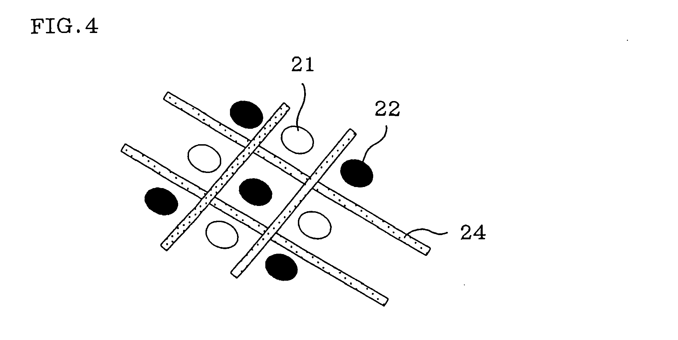

Figure 1 is a perspective view schematically showing an embodiment of the present invention. Figure 2 is an enlarged view of part of Figure 1 showing that the distance between spot centers (spot pitch) of the same reagent (L2) is greater than the distance between spot centers (spot pitch) of the two different reagents (L1). Figure 3 is a diagram schematically showing an embodiment of the present invention with shields disposed between the reagent spots on the surface and inside of the support. Figure 4 is a diagram schematically showing that the shields are linear.

### BEST MODE FOR CARRYING OUT THE INVENTION

An embodiment of the analytical test piece of the present invention will now be specifically explained with reference to the drawings.

As shown in Figure 1, the analytical test piece 10 of this embodiment has a support 1 and, reagent spots 2, which are aligned on the surface 11 of the support 1 or on the surface 11 and the inside of the support 1 in a specified spot alignment pattern (a spot pitch). An analyte-containing sample 3 introduced onto the surface 11 of the support 1 comes in contact with and reacts with the reagent spots 2 aligned on the surface 11 of the support 1 or on the surface 11 and inside of the support 1 and produces a detectable substance (a signal substance) or exhibits a detectable property (a signal property). There are two or more types of reagent spots (indicated as 21 and 22 in Figure 1) in the reagent spots 2, with each reagent spot containing an ingredient which exhibits a given function when mixed with the other ingredients. The sample 3 introduced to the surface 11 of the support 1 comes in contact with the two or more types of reagent spots 21 and 22, causes the reagent spots 21 and 22 to mix together, and reacts with the mixed reagent spots, thereby forming a signal substance or exhibiting a signal property.

The analytical reliability, analytical sensitivity (analytical precision), and storage stability can be increased in this manner.

Any material having a surface 11 can be used as the support 1 in this embodiment without any specific limitations. A porous material can be given as a preferred example. A hydrophilic porous material is particularly preferable. Celluloses, polyether sulfones, acrylic polymers, and the like having pores with a pore diameter from 0.2 µm to several µm are preferably used. The use of a porous material as the support 1 increases the amount of the content in the solution in the reagent spots 2 (21, 22) to be permeated into the support 1, which results in improved analytical sensitivity.

As the two or more types of solutions for the reagent spots 2 (reagent spots 21 and 22) used in this embodiment, any compounds that can be mixed with each other by introducing the sample 3 and produce a signal substance or exhibit a signal property by being brought into contact with and reacting with the analyte in the sample 3 may be used without any specific limitations. For example, when the activity of lactate dehydrogenase as an analyte is measured (i.e. when a solution containing lactate dehydrogenase is used as an analyte), a solution containing lactic acid as a substrate, NAD (nicotinamide adenine dinucleotide) as a coenzyme, 1-methoxy PMS (phenazine methosulfate) as an electron carrier, and NTB (nitrotetrazolium blue) as a tetrazolium reagent is used as the solution for one reagent spot 21, and a buffer solution such as a phosphate buffer, Tris hydrochloric acid (Tris-HCl) buffer, or the like is used as the solution for another reagent spot 22.

In this embodiment, at least one of the solutions for the reagent spots 21 and 22 preferably contains a coloring substance. The coloring substance ensures production of a manifest signal substance or exhibition of a manifest signal property. As examples of the solution containing such a coloring substance, reducing reagents such as a Formazan reagent and oxidizing reagents such as a 4-aminoantipyrine solution, a phenol solution, and the like can be mentioned.

At least one of the solutions for the reagent spots 2 (reagent spots 21 and 22) preferably contains a fluorescent substance. The fluorescent substance ensures a fluorescent inspection of contact of the reagent spots 21 and 22 with each other which is difficult to confirm with naked eye inspection and thereby ensures stable inspection quality. Although there are no specific limitations, a substance that can emit fluorescence without impairing inspection performance of the reagent spots 21 and 22, for example, food colors of green, yellow, blue, and the like can be given.

In addition, at least one of the solutions for the reagent spots 2 (reagent spots 21 and 22) preferably contains a water-soluble polymer. The water-soluble polymer prevents the components of the reagent spots 21 and 22 from leaking out due to moisture in the atmosphere and ensures stable storage of the reagent spots 21 and 22 for a long period of time without impairing the properties of the reagents.

As shown in Figure 3, water-soluble shields 24 are preferably located in the spaces where no reagent spots 21 and 22 are disposed (spaces between reagent spots) on the surface 11 of the support 1 or on the surface 11 and inside of the support 1 to ensure long-term storage stability. In this instance, the water-soluble shield 24 is preferably located at a point from which the distance to a reagent spot 21 and a reagent spot 22 is the smallest. A shield 24 with any dimension that does not contact the reagent spots 21 and 22 may be used. The shield of any shape which can be located between the reagent spots 21 and 22 without coming into contact with them can be used without any specific limitations. To increase long term stability, a straight line shield shown in Figure 4 is preferable. However, a curved shield is also acceptable.

Although there are no specific limitations to the material of the shield 24, water-soluble polymers and the like can be given as examples. A water-soluble polymer can maximize the region of the reagent spots 21 and 22 and increase the sensitivity, because the water-soluble polymer does not excessively expand on the surface and inside of the support due to the comparatively high viscosity. As preferable examples of the water-soluble polymer used for forming the shield 24, sugar polymers (e.g. pullulan), polypropylene glycol, polyethylene glycol, sodium carboxycellulose, polyvinyl alcohol, dextran, partial hydrolyzate of starch, and the like can be given. Although there are no specific limitations, the method for forming a shield 24 may be appropriately selected according to the degree of hydrophilicity of the support 1. For example, when a support 1 with comparatively high hydrophilicity is used, a screen printing method which can easily cause the shield 24 to permeate the inside of the support 1 is preferably used. When a support 1 with comparatively low hydrophilicity, which causes the shield 24 to permeate the inside thereof only with difficulty, is used, the inkjet method is preferably used to ensure permeation of the shield material in the thickness direction. Of these, the inkjet method is more preferable due to the capability of increasing the sensitivity by producing a minute pattern of the shields 24 and enlarging the area in which the reagent spots 21 and 22 are formed.

The reagent spots 2 (reagent spots 21 and 22) aligned in at least one area on the surface 11 of the support 1 or on the surface 11 and inside of the support 1 are preferably a different type from the reagent spots 2 (reagent spots 21 and 22) aligned in other areas. This manner of alignment of the reagent spots 2 enables inspection of two or more items using a small amount of sample.

The reagent spots 2 (reagent spots 21 and 22) aligned in at least one area on the surface 11 of the support 1 or on the surface 11 and inside of the support 1 preferably have a concentration different from the reagent spots 2 (reagent spots 21 and 22) aligned in other areas. This manner of alignment of the reagent spots 2 enables quantitative analysis using one piece of analytical test specimen.

When the reagent spots 2 (reagent spots 21 and 22) are aligned not only on the surface, but also inside the support 1, the reagent spots 2 (reagent spots 21 and 22) are preferably aligned so that those on the surface 11 of the support 1 exhibit the highest reactivity with the samples. This manner of alignment of the reagent spots 2 enables exhibition of a clear signal property with a small amount of sample and ensures unfailimg inspection.

When the reagent spots 2 (reagent spots 21 and 22) are aligned not only on the surface, but also inside the support 1, the reagent spots 2 (reagent spots 21 and 22) are preferably aligned so that those on the surface 11 of the support 1 have the highest concentration. This manner of alignment of the reagent spots 2 enables exhibition of clear signal property with a small amount of sample and ensures unfailing inspection.

A surfactant is preferably provided in the spaces where no reagent spots 2 (reagent spots 21 and 22) are aligned (between reagent spots) on the surface 11 of the support 1 or on the surface 11 and inside of the support 1. The surfactant can reduce the time of contact and reaction of the reagent spots 21 and 22 with the sample 3 and accelerate dispersion of signal substances. As the surfactant, any of anionic surfactants, cationic surfactants, and nonionic surfactants may be appropriately selected according to the use conditions. As preferable examples, anionic surfactants such as alkylaryl sulfonate and alkylbenzene sulfonate, cationic surfactants such as alkyltrimethyl ammonium and alkyl pyridinium, nonionic surfactants such as polyoxyethylene fatty acid ester, polyoxyethylene alkyl phenyl, and the like can be given.

A foaming agent is preferably provided in the spaces where no reagent spots 2 (reagent spots 21 and 22) are aligned (between reagent spots) on the surface 11 of the support 1 or on the surface 11 and inside of the support 1. The foaming agent reduces the time for the contact and reaction of the reagent spots 21 and 22 with the sample 3 by promoting mixing of the reagent spot 21 and reagent spot 22. As such a foaming agent, separately disposed potassium hydrogencarbonate (KHCO₃) or sodium hydrogencarbonate (NaHCO₃) and an organic acid can be given, for example.

It is desirable to provide a supporting body 4 for supporting the support 1 on the opposite surface (back) to the surface 11 of the support 1. The supporting body 4 ensures easy operation and easy alignment of the reagent spots 2 (reagent spots 21 and 22). The supporting body 4 may have any configuration that can surely support the support 1 without specific limitation. As the material for the supporting body 4, a metal, ceramic, glass, resin, and the like can be mentioned. Of these, resins are preferable from the viewpoint of the low cost and stability to solutions. As examples of the resin, PET resins, acrylic resins, vinyl chloride resins, and the like can be given.

The cross-section of the reagent spots 21 and 22 cut along a specified plane parallel to the surface 11 of the support 1, for example, a plane at a 1/2 depth from the surface 11 of the support 1, is preferably oval or elliptical, or has the shape of a race track. Such a cross-sectional configuration can reduce the time for the contact and reaction of the reagent spots 21 and 22 with the sample 3.

As shown in Figure 2, the distance between spot centers (spot pitch) of the same reagent (in Figure 2, a reagent spot 21 and another reagent spot 21 or a reagent spot 22 and another reagent spot 22) (L2) is preferably greater than the distance between spot centers (spot pitch) of the two different reagents (Figure 2, a reagent spot 21 and a reagent spot 22) (L1). Such an arrangement ensures mixing of the reagent spots 21 and 22 when the sample 3 is introduced.

When the diameter of the reagent spots 21 and 22 is preferably 0.5 mm or less, and more preferably 0.05 to 0.25 mm, and the distance (spot pitch) between the center of the different reagent spots (a reagent spot 21 and a reagent spot 22) (L1) is preferably 0.6 mm or less, and more preferably 0.1 to 0.4 mm, as shown in Figure 2, the time for the contact and reaction of the reagent spots 21 and 22 with the sample 3 can be reduced and dispersion of signal substances is promoted and, at the same time, the sample 3, the reagent spot 21 and reagent spot 22 can be homogeneously mixed, whereby production of the signal substances and exhibition of the signal property can be ensured. The above reagent spot diameter and spot pitch are particularly preferable when the inspection and analysis rely upon the amount of luminescence as a signal property, because a luminous intensity can be easily identified by naked eye observation.

In aligning the reagent spots 21 and 22 on the surface 11 of the support 1 or on the surface 11 and inside of the support 1, it is desirable that the alignment ratio (A) = (S1/S2) satisfy the inequality 0.01 < (A) < 0.814, wherein S 1 indicates the total cross-sectional areas of the reagent spots 21 and 22 in a specified plane parallel to the surface 11 of the support 1, for example, a plane at a 1/2 depth from the surface 11 of the support 1, and S2 is the area of the surface 11 of the support 1. If the alignment ratio (A) is 0.01 or less, it is difficult to reduce the time for the contact and reaction of the reagent spots 21 and 22 with the sample 3; if 0.814 or more, long term stability may be impaired. Taking the long term reliability into consideration, a more preferable range for the alignment ratio (A) is 0.09 < (A) < 0.35.

It is further preferable that a spot pitch, which is the aligning pattern of the reagent spots 21 and 22 on the surface 11 of the support 1 or on the surface 11 and inside of the support 1, in a certain area differs from the spot pitch in other areas. Since the reagent spots 21 and 22 in the area with a short spot pitch deteriorates due to absorption of water faster than in other areas, it is easy to determine quantitatively deterioration of the reagent spots 21 and 22 by differentiating the spot pitch in a certain area from the other areas. In addition, differentiating the spot pitch enlarges the analytical (measuring) area.

It is further desirable to provide confirmation reagent spots outside the area in which the reagent spots 21 and 22 are aligned according to a specified pattern for identifying proper alignment of spots on the surface of the support 1 or on the surface and in the inside of the support 1. Providing such additional reagent spots makes it possible to confirm mixing conditions of a reagent spot 21 and a reagent spot 22, for example, before aligning the reagent spots 21 and 22, thereby increasing the reliability of the analytical test piece 10.

In this instance, each of the confirmation reagent spots 23 preferably contains the above-described different solution. In this manner, production of a signal substance and exhibition of a signal property can be promptly confirmed beforehand, whereby the reliability of the test piece can be increased.

The amount of the solution (spot amount) in the reagent spots 21 and 22 in at least one area on the surface 11 of the support 1 or on the surface 11 and inside of the support 1 preferably differs from the spot amount in other areas, for example, the spot amount in the central area to which the sample 3 is introduced is larger than the spot amount of other areas. In this manner, an optimum spot amount range for the sample 3 can be expanded, that is, the analytical sensitivity can be easily altered, so that quantitative analysis is possible using one sheet of analytical test piece.

Although the analytical test piece of the present invention is preferably prepared by the inkjet method as mentioned later, the needle method of forming a reagent spot, in which a needle is caused to come in contact with a flat plane of the support to transfer the solution to the flat plane, can also be used. There are various types of needle tips. Needles having any type of needle tip, such as a solid needle type with no grooves at the tip, a quill needle type with a fountain pen groove at the tip, a needle-and-ring type with a ring to store a solution of which the membrane is pierced by a needle to cause the solution to pour out, and the like can be used. Of these, the quill needle type is preferable.

An embodiment for preparing the analytical test piece of the present invention will now be specifically explained with reference to the drawings.

As shown in Figure 1, the method for preparing the analytical test piece 10 of this embodiment comprises forming reagent spots 2 by aligning prescribed solutions on the surface 11 of the support 1 or on the surface 11 and inside of the support 1 in a prescribed spot alignment pattern (a spot pitch), causing an analyte-containing sample 3 introduced onto the surface 11 of the support 1 to come in contact with and react with the reagent spots 2, thereby producing a detectable substance (a signal substance) or exhibiting a detectable property (a signal property), wherein two or more types of reagent spots (shown by 21 and 22 in Figure 1), each containing a solution different from the solution in the other types of spots and each exhibiting a given function by being mixed together with the other types of spots when brought into contact with a sample 3 to be introduced, are formed on the surface 11 of the support 1 or on the surface 11 and the inside of the support 1, by means of the inkjet method described later, and the sample 3 introduced onto the surface 11 of the support 1 comes in contact with the reagent spots 21 and 22 formed on the surface 11 of the support 1 or on the surface 11 and inside of the support 1, causes the reagent spots 21 and 22 to mix, and reacts with the mixed reagent spots 21 and 22 to produce a signal substance or exhibit a signal property.

Providing such spots containing two or more different types of solutions ensures production of the analytical test piece 10 with excellent storage stability. In addition, the inkjet method can accurately and densely locate spots, whereby a precise amount of reagent can be charged into each reagent spot. Consequently, not only the sensitivity distribution in the analytical test piece can be minimized, which results in increased analytical reliability, but also uniform analytical quality can be ensured even if a large analytical test piece is used, which gives rise to increased production efficiency. In addition, a high sensitive analytical test piece capable of analyzing a very small amount of samples can be obtained by densely locating the reagent spots. Furthermore, since a precise amount of reagent can be charged into the reagent spots, the storage stability of the analytical test piece can be improved.

As the liquid drop injector used for the inkjet method of this embodiment, an apparatus comprising a fluid channel substrate in which a fluid channel is formed, an actuator installed in the fluid channel substrate having a function of changing the volume of a cavity as a pressurizing chamber, a nozzle substrate attached to the bottom of the fluid channel substrate with nozzles formed therein, and a liquid receiver installed on the rear top of the fluid channel substrate can be given, for example. Specifically, the apparatus disclosed in Japanese Patent Application Laid-open No. 2003-75305, for example, can be preferably used.

As the system for controlling this liquid drop injector, a system described in Japanese Patent Application Laid-open No. 2003-98183, for example, can be preferably used.

Any material having a surface 11 can be used as the support 1 in this embodiment without any specific limitations. A porous material can be given as a preferred example. A hydrophilic porous material is particularly preferable. Celluloses, polyether sulfones, acrylic polymers, and the like having pores with a pore diameter from 0.2 µm to several µm are preferably used. The use of a porous material as the support 1 increases the amount of the solution in the obtained reagent spots 2 (21, 22) to permeate the support 1, which results in improved analytical sensitivity. Since the inkjet method can supply reagents to the support without contact, an analytical test piece with no surface unevenness can be obtained even if a porous material is used as the support.

As the two or more types of solutions for the reagent spots 2 (reagent spots 21 and 22) used in this embodiment, any compounds that can be mixed with each other by introducing the sample 3 and produce a signal substance or exhibit a signal property by being brought into contact with and reacting with the analyte in the sample 3 may be used without any specific limitations. For example, when the activity of lactate dehydrogenase as an analyte is measured (i.e. when a solution containing lactate dehydrogenase is used as an analyte), a solution containing lactic acid as a substrate, NAD (nicotinamide adenine dinucleotide) as a coenzyme, 1-methoxy PMS (phenazine methosulfate) as an electron carrier, and NTB (nitrotetrazolium blue) as a tetrazolium reagent is used as the solution for one reagent spot 21, and a buffer solution such as a phosphate buffer, Tris hydrochloric acid (Tris-HCl) buffer, or the like is used as the solution for another reagent spot 22.

In this embodiment, at least one of the solutions for the reagent spots 21 and 22 preferably contains a coloring substance. An analytical test piece 10 capable of producing a manifest signal substance or exhibiting a manifest signal property can be obtained by using a coloring substance. As examples of the solution containing such a coloring substance, reducing reagents such as a Formazan reagent and oxidizing reagents such as a 4-aminoantipyrine solution, a phenol solution, and the like can be mentioned. In this instance, the inkjet method can be used for supplying a solution containing a coloring substance. The inkjet method can supply a precise quantity of a coloring substance and produce a high quality analytical test piece.

Two or more solutions in the reagent spots 21 and 22 preferably contains a fluorescent substance. The fluorescent substance ensures a fluorescent inspection of contact of the reagent spots 21 and 22 with each other which is difficult to confirm with naked eye inspection and thereby ensures stable inspection quality. Although there are no specific limitations, a substance that can emit fluorescence without impairing inspection performance of the reagent spots 21 and 22, for example, food colors of green, yellow, blue, and the like can be given. In this instance, in the same manner as in the case of the coloring substance, the inkjet method can be used for supplying a solution containing a fluorescent substance. The inkjet method can supply a precise quantity of a fluorescent substance and produce a high quality analytical test piece.

Adding a water-soluble polymer to at least one of the solutions is preferable from the viewpoint of ensuring long term storage stability. The water-soluble polymer prevents the components of the reagent spots 21 and 22 from escaping to the atmosphere due to moisture in the atmosphere and ensures stable storage of the reagent spots 21 and 22 for a long period of time without impairing the properties of the reagents.

As shown in Figure 3, water-soluble shields 24 are preferably located in the spaces where no reagent spots 21 and 22 are disposed (spaces between reagent spots) on the surface 11 of the support 1 or on the surface 11 and inside of the support 1 to ensure stable storage of the reagent spots 21 and 22 for a long period of time. In this instance, the shield 24 is preferably located at a point from which the distance to a reagent spot 21 and a reagent spot 22 is the smallest. A shield 24 with any dimension that does not contact the reagent spots 21 and 22 may be used. A shield of any shape which can be located between the reagent spots 21 and 22 without coming into contact with them can be used without any specific limitations. To increase long term stability, a straight line shield shown in Figure 4 is preferable. However, a curved shield is also acceptable.

Although there are no specific limitations to the material of the shield 24, water-soluble polymers and the like can be given as examples. A water-soluble polymer can maximize the region of the reagent spots 21 and 22 and increase the sensitivity, because the water-soluble polymer does not excessively expand on the surface and inside of the support due to the comparatively high viscosity. As preferable examples of the water-soluble polymer used for forming the shield 24, sugar polymers (e.g. pullulan), polypropylene glycol, polyethylene glycol, sodium carboxycellulose, polyvinyl alcohol, dextran, partial hydrolyzate of starch, and the like can be given. Although there are no specific limitations, the method for forming the shield 24 may be appropriately selected according to the degree of hydrophilicity of the support 1. For example, when a support 1 with comparatively high hydrophilicity is used, a screen printing method which can easily cause the shield 24 to permeate the inside of the support 1 is preferably used. When a support 1 with a comparatively low hydrophilicity, which causes the shield 24 to permeate the inside thereof only with difficulty, is used, the inkjet method is preferably used to ensure permeation of the shield material in the thickness direction. Of these, the inkjet method is more preferable due to the capability of increasing the sensitivity by producing a minute pattern of the shield 24 and enlarging the area in which the reagent spots 21 and 22 are formed.

The cross-section of the reagent spots 21 and 22 cut along a specified plane parallel to the surface 11 of the support 1, for example, a plane at a 1/2 depth from the surface 11 of the support 1, is preferably oval or elliptical, or has the shape of a race track. This configuration makes it possible for the analytical test piece to reduce the time for the contact and reaction of the reagent spots 21 and 22 with the sample 3. The above-described configuration can be easily formed by using the inkjet method.

As shown in Figure 2, the distance between spot centers (spot pitch) of the same reagent (in Figure 2, a reagent spot 21 and another reagent spot 21 or a reagent spot 22 and another reagent spot 22) (L2) is preferably greater than the distance between spot centers (spot pitch) of the two different reagents (Figure 2, a reagent spot 21 and a reagent spot 22) (L1). Such an arrangement ensures mixing of the reagent spots 21 and 22 when the sample 3 is introduced.

When the diameter of the reagent spots 21 and 22 is preferably 0.5 mm or less, and more preferably 0.05 to 0.25 mm, and the distance (spot pitch) between the center of the different reagent spots (a reagent spot 21 and a reagent spot 22) (L1) is preferably 0.6 mm or less, and more preferably 0.1 to 0.4 mm, as shown in Figure 2, the analytical test piece 10 which can reduce the time for the contact and reaction of the reagent spots 21 and 22 with the sample 3 and also can ensure homogeneous mixing of the sample 3, the reagent spot 21 and reagent spot 22, thereby ensuring production of the signal substances and exhibition of the signal property, can be obtained. The above reagent spot diameter and spot pitch are particularly preferable when the inspection and analysis rely upon the amount of luminescence as a signal property, because a luminous intensity can be easily identified by naked eye observation.

In aligning the reagent spots 21 and 22 on the surface 11 of the support 1 or on the surface 11 and inside of the support 1, it is desirable that the alignment ratio (A) = (S1/S2) satisfy the inequality 0.01 < (A) < 0.814, wherein S1 indicates the total cross-sectional areas of the reagent spots 21 and 22 in a specified plane parallel to the surface 11 of the support 1, for example, a plane at a 1/2 depth from the surface 11 of the support 1, and S2 is the area of the surface 11 of the support 1. If the alignment ratio (A) is 0.01 or less, it is difficult to obtain an analytical test piece 10 which can reduce the time for the contact and reaction of the reagent spots 21 and 22 with the sample 3; if 0.814 or more, long term stability of the analytical test piece 10 may be impaired. Taking the long term reliability of the analytical test piece 10 into consideration, a more preferable range for the alignment ratio (A) is 0.09 < (A) < 0.35. The above spot pitch, diameter, and cross-sectional area alignment ratio can be efficiently achieved by using the inkjet method which can supply a precise amount of reagent without contact.

It is further preferable that a spot pitch, which is the aligning pattern of the reagent spots 21 and 22 on the surface 11 of the support 1 or on the surface 11 and inside of the support 1, in a certain area differs from the spot pitch in other areas. Since the reagent spots 21 and 22 in the area with a short spot pitch deteriorates due to absorption of water faster than in other areas, it is possible to obtain an analytical test piece 10 which can quantitatively determine deterioration of the reagent spots 21 and 22 with ease by differentiating the spot pitch in a certain area from the other area. In addition, differentiating the spot pitch can produce an analytical test piece 10 with an enlarged analytical (measuring) area. In addition, this manner of alignment of the reagent spots 2 enables quantitative analysis using one piece of analytical test specimen.

It is further desirable to provide confirmation reagent spots 23 for identifying proper alignment of spots outside the area in which the reagent spots 21 and 22 are aligned according to a specified pattern on the surface of the support 1 or on the surface and inside of the support 1. In this instance, the confirmation reagent spots 23 for identifying proper alignment may be located within the ultimate configuration of analytical test piece 10 or may be located on the support 1 before the ultimate configuration is formed (outside the analytical test piece 10). Providing such additional reagent spots makes it possible to confirm mixing conditions of a reagent spot 21 and a reagent spot 22, for example, before aligning the reagent spots 21 and 22, thereby increasing the reliability of the analytical test piece 10.

In this instance, each of the confirmation reagent spots 23 preferably contains the above-described different solution. In this manner, production of a signal substance and exhibition of a signal property can be promptly confirmed beforehand, whereby the reliability of the test piece can be increased. If the inkjet method is used for forming the confirmation reagent spots 23, the same spots as the reagent spots 21 and 22 in the analytical test piece 10 can be easily formed, whereby precise inspection can be ensured.

The reagent spots 21 and 22 aligned in at least one area on the surface 11 of the support 1 or on the surface 11 and inside of the support 1 are preferably a different type from the reagent spots 21 and 22 aligned in other areas. This manner of alignment of the reagent spots 2 enables inspection of two or more items using a small amount of sample. In addition, since reagent spots can be formed at a high density by using the inkjet method, it is possible to prepare chips capable of inspecting different types of substances without enlarging the size of the chips.

The reagent spots 21 and 22 aligned in at least one area on the surface 11 of the support 1 or on the surface 11 and inside of the support 1 preferably have a different concentration from the reagent spots 21 and 22 aligned in other areas. Since reagent spots can be formed at a high density by using the inkjet method, it is possible to change the concentration even when the area is small. This manner of alignment of the reagent spots 2 enables quantitative analysis using one piece of analytical test specimen.

When the reagent spots 21 and 22 are aligned not only on the surface, but also inside the support 1, the reagent spots 21 and 22 are preferably aligned so that those on the surface 11 of the support 1 exhibit the highest reactivity with samples. This manner of alignment of the reagent spots 2 enables exhibition of a clear signal property with a small amount of sample and ensures unfailing inspection. By using the inkjet method, the reagent spots can be aligned so that those on the surface 11 exhibit the highest reactivity with samples. Specifically, such reagent alignment is efficiently achieved by providing a solution with a specified concentration injected in a certain area of the support 1, and then injecting the solution with a larger concentration in the same area using the same apparatus with a longer drying time at the nozzle plane that is , for example, with a longer interval time between each injection.

When the reagent spots 21 and 22 are aligned not only on the surface, but also inside the support 1, the reagent spots 21 and 22 are preferably aligned so that those on the surface of the support 1 have a highest concentration. This manner of alignment of the reagent spots 2 enables exhibition of a clear signal property with a small amount of sample and ensures unfailing inspection.

A surfactant (not shown) is preferably provided in the spaces where no reagent spots 21 and 22 are aligned (between the reagent spots 21 and 22) on the surface 11 of the support 1 or on the surface 11 and inside of the support 1. The use of the surfactant can produce an analytical test piece 10 which can reduce the time for the contact and reaction of the reagent spots 21 and 22 with the sample 3 and accelerate dispersion of signal substances. As the surfactant, any of anionic surfactants, cationic surfactants, and nonionic surfactants may be appropriately selected according to the use conditions. As preferable examples, anionic surfactants such as alkylaryl sulfonate and alkylbenzene sulfonate, cationic surfactants such as alkyltrimethyl ammonium and alkyl pyridinium, nonionic surfactants such as polyoxyethylene fatty acid ester, polyoxyethylene alkyl phenyl, and the like can be given.

A foaming agent (not shown) is preferably provided in the spaces where no reagent spots 21 and 22 are aligned (between the reagent spots 21 and 22) on the surface 11 of the support 1 or on the surface 11 and inside of the support 1. The use of the foaming agent can provide an analytical test piece 10 which can reduce the time for the contact and reaction of the reagent spots 21 and 22 with the sample 3 by promoting mixing of the reagent spot 21 and reagent spot 22. As such a foaming agent, separately disposed potassium hydrogencarbonate (KHCO₃) or sodium hydrogencarbonate (NaHCO₃) and an organic acid can be given, for example. Since the inkjet method can uniformly supply a very small amount of surfactant and foaming agent, a large number of analytical test pieces 10 with uniform quality can be prepared.

It is desirable to provide a supporting body 4 for supporting the support 1 on the opposite surface (back) to the surface 11 of the support 1. The supporting body 4 ensures easy operation and easy alignment of the reagent spots. The supporting body 4 may have any configuration that can surely support the support 1 without specific limitation. As the material for the supporting body 4, a metal, ceramic, glass, resin, and the like can be mentioned. Of these, resins are preferable from the viewpoint of the low cost and stability to solutions. As examples of the resin, PET resins, acrylic resins, vinyl chloride resins, and the like can be given.

In injecting several solutions for aligning reagent spots 21 and 22 using the inkjet method, it is preferable to inject the solutions in a direction other than the vertical direction to the surface 11 of the support 1. Such a manner of injection can provide the liquid drops injected from the nozzles with a longer time for arriving at the support 1 and supply liquid drops with a higher concentration, whereby analytical sensitivity of the resulting analytical test piece 10 can be increased.

A great many very small independent droplets can be preferably produced by using the inkjet method. Since such a great many very small independent droplets are concentrated before reaching the support 1, it is possible to supply the reagent spots 21 and 22 with a large spot amount (the amount of liquid drops forming the reagent spots 21 and 22) without enlarging the spot diameter of the reagent spots 21 and 22, whereby analytical sensitivity of the resulting analytical test piece 10 can be further increased.

The amount of liquid drops forming the reagent spots 21 and 22 (spot amount) is preferably 0.1 µl or less, and more preferably 0.002 to 0.02 µl. If more than 0.1 µl, the liquid drops of the solution expand on the support 1 and unnecessarily enlarge the spot diameter, resulting in a analytical test piece 10 which possibly takes a long time to cause the reagent spots 21 and 22 to come into contact and react with the sample 3.

The amount of the solution (spot amount) in the reagent spots 21 and 22 in at least one area on the surface 11 of the support 1 preferably differs from the spot amount in other areas, for example, the spot amount in the central area to which the sample 3 is introduced is larger than the spot amount of other areas. In this manner, an optimum spot amount range for the sample 3 can be expanded, that is, the analytical sensitivity of the resulting analytical test piece 10 can be easily altered, so that quantitative analysis is possible using one sheet of the analytical test piece 10. In addition, even small chips can be easily formed by using the inkjet method.

Each of the reagent spots 21 and 22 is preferably formed by injecting droplets of the solution two or more times. In this manner, the spot amount can be increased and thereby the sensitivity of the resulting analytical test piece 10 can be increased without unnecessarily enlarging the spot diameter.

In forming the reagent spots 21 and 22, the liquid drops of the solution are injected onto the support 1 which is maintained preferably at 40°C or less, more preferably 15 to 30°C to ensure quick drying of the liquid drops after forming the reagent spots 21 and 22, while suppressing expansion of the reagent spots 21 and 22. In this manner, the spot amount can be increased without unnecessarily enlarging the spot diameter and the sensitivity of the resulting analytical test piece 10 can be increased.

In addition, the liquid drops of the solution are preferably injected while the back of the support 1 is separated from the supporting body 4. Because this manner of forming the reagent spots 21 and 22 can suppress undue expansion of the reagent spots 21 and 22 on the back of the support 1 and can prevent unnecessary mixing of the liquid drops, the sensitivity of the resulting analytical test piece 10 can be increased.

The analytical test piece 10 prepared by any of the above-described methods is provided with a support 1 with a surface 11 and reagent spots 2 consisting of reagent spots 21 and 22 which are aligned on the surface 11 of the support 1 or on the surface 11 and the inside of the support 1 in a specified spot alignment pattern (a spot pitch), the reagent spots being formed by the inkjet method by injecting liquid drops of two or more different solutions, each exhibiting a given function upon being mixed together, wherein an introduced analyte-containing sample 3 comes in contact with the reagent spots 21 and 22, causes the reagent spots 21 and 22 to mix together, and reacts with the mixed reagent spots, thereby forming a detectable substance or detectable property (a signal substance or signal property). The analytical test piece is useful as an inspection chip for inspecting and analyzing samples 3 containing an analyte (e.g. a body fluid, particularly urine, blood, etc. of humans and animals) introduced onto the surface 11 of the support 1 and excelling in analysis reliability, analytical sensitivity (analytical precision), and storage stability.

### EXAMPLES

The present invention will now be described in more detail by way of examples.

(Examples 1 to 2)
Sample analytical test pieces were prepared by using a needle method for forming reagent spots according to the following procedure and were subjected to evaluation of long term stability and the other properties. As a first reagent, a first solution containing lactic acid, NAD (nicotinamideadeninedinucleotide), 1-methoxy PMS (phenazine methosulfate), and NTB (nitrotetrazolium blue) was used. As a second reagent, a phosphate buffer (a second solution) was used. Cow milk was used as an analyte sample. As supports, 5 mm × 5 mm boards with a thickness of 0.16 mm made of hydrophilic cellulose mixture ester with pore size of 0.8 µm were used.

Using the above-mentioned first solution, second solution, supports, and the needle method (the method of using a quill needle of the type with a groove like the groove in a fountain pen) as the method of forming reagent spots, the first solution and the second solution were carried on the needle, which was brought into contact with the supports to dispense the first and second solutions onto the supports to form fist and second reagent spots. In this instance, the first and second solutions in approximate amounts of 0.01 µl and 0.06 µl were respectively dispensed to each of the first and second reagent spots to form the first and second reagent spots with approximate pitches of 0.6 mm and 1.5 mm, and approximate diameters of 0.3 mm and 1.2 mm. Properties of the resulting first and second samples were evaluated. The results are shown in Table 1.

(Comparative Example 1)
A comparative analytical test piece sample was prepared using the liquid impregnation method as the method for forming reagent spots. Properties of the resulting sample were evaluated. Specifically, the same first and second reagents as used in Example 1 were put into beakers. Then, the same support material as used in Example 1 was put into the beaker containing the first reagent to impregnate the support with the first reagent. After drying at 30°C, the support material was put into the beaker containing the second reagent to impregnate the support with the second reagent. The resulting support material was dried at 30°C to obtain the comparative sample impregnated with the first and second reagents. Properties of the obtained comparative sample were evaluated. The results are shown in Table 1.

Properties were evaluated in Examples 1 and 2 and Comparative Example 1 after storing the samples for one day, seven days, and 30 days after preparation at 4°C by irradiating the entire central area of the surface of the samples with a laser beam at a wavelength of 520 nm and measuring the reflectance. The higher the reflectance (i.e. the smaller the change in luminance), the better the stability. The case in which the reflectance decreased 10% or more from the initial reflectance (reflectance immediately after preparation) in all samples was rated as "Bad" and the case in which all the samples exhibited a reflectance decrease of less than 10% was rated as "Good". In Example 2, the first and second reagents exhibited unevenness in the concentration in the central area and the neighborhood of the reagent spots. However, the unevenness presented no problem in practice. An analyte sample was dropped onto each of the above analytical test piece samples to measure the reflectance in the same manner as above to confirm the sensitivity. The sensitivity was rated as "Good" only in the case in which all samples after dropping the analyte samples exhibited a reflectance decrease of 20% or more from the reflectance before dropping the analyte samples. In addition, the samples were observed by the naked eye for inspection of unevenness in spots and unevenness in a 5 mm × 5 mm square.

**TABLE 1**

| | | Comparative Example | Example 1 | Example 2 |
|---|---|---|---|---|
| Pitch (mm) | | - | 0.6 | 1.5 |
| (Reagent spot forming method) | | (Impregnation method) | (Needle method) | (Needle method) |
| Diameter (mm) (Reagent spot) | | - | 0.3 | 1.2 |
| Spot amount (µl) | | - | 0.01 | 0.06 |
| Stability | | | | |
| | 1 day after preparation | Good | Good | Good |
| | 7 days after preparation | Good | Good | Good . |
| | 30 day after preparation | Bad | Good | Good |
| Unevenness in spots (Sensitivity) | | None (Good) | None (Good) | None (Good) |
| Unevenness in chips | | None | None | None |

As shown in Table 1, it was confirmed that the reagent spots formed by aligning specific solutions (the first and second solutions in Examples 1 and 2) on the surface and inside of the support in a prescribed spot alignment pattern (a spot pitch) can be brought into contact and react with an analyte-containing sample introduced onto the surface of the support, and produce a detectable signal. It was further confirmed that an analytical test piece excelling in long term stability can be obtained. Although two reagent solutions were used in Examples 1 and 2, taking stability into consideration, three or four types of reagent solutions may be used for forming the reagent spots.

(Examples 3 to 5)
Samples were prepared by using the inkjet method for forming reagent spots according to the following procedure and subjected to evaluation of long term stability and other properties. As a first reagent, a first solution containing lactic acid, NAD (nicotinamideadeninedinucleotide), 1-methoxy PMS (phenazine methosulfate), and NTB (nitrotetrazolium blue) was used. As a second reagent, a phosphate buffer (a second solution) was used. Cow milk was used as an analyte sample. As supports, 5 mm × 5 mm boards with a thickness of 0.16 mm made of hydrophilic cellulose mixture ester with pore size of 0.8 µm were used. First and second reagent spots were prepared using the first and second solutions, the supports, and the injecting unit described in Japanese Patent Application Laid-open No. 2003-75305. In this instance, the first and second solutions in approximate amounts of 0.003 µl, 0.01 µl, and 0.06 µl were respectively dispensed to each of the first and second reagent spots to form the first and second reagent spots with approximate pitches of 0.3 mm, 0.6 mm, and 1.5 mm, and approximate diameters of 0.15 mm, 0.3 mm and 1.2 mm. Properties of the resulting first to third samples (Examples 3 to 5) were evaluated. The results are shown in Table 2.

(Comparative Example 2)
A first comparative sample (Comparative Example 2) was prepared using the liquid impregnation method as the method for forming reagent spots. Properties of the resulting sample were evaluated. Specifically, the same first and second reagents as used in Example 3 were put into beakers. Then, the same support material as used in Example 3 was put into the beaker containing the first reagent to impregnate the support with the first reagent. After drying at 30°C, the support material was put into the beaker containing the second reagent to impregnate the support with the second reagent. The resulting support material is dried at 30°C to obtain a comparative sample impregnated with the first and second reagents. Properties of the obtained first comparative sample (Comparative Example 2) were evaluated. The results are shown in Table 2.

(Comparative Example 3)
The second comparative sample (Comparative Example 3) was prepared using the needle method of forming reagent spots, in which the first solution and the second solution were carried on the needle, which was brought into contact with the support to dispense the first and second solutions onto the support to form first and second reagent spots. A quill needle of the type with a groove like the groove in a fountain pen was used as the needle. The results are shown in Table 2. In this instance, the first and second solutions in an approximate amount of 0.003 µl were dispensed to each of the first and second reagent spots to form the first and second reagent spots with an approximate pitch of 0.3 mm, and approximate diameter of 0.15 mm. Properties of the resulting second comparative sample (Comparative Example 3) were evaluated. The results are shown in Table 2.

Properties were evaluated in Examples 3 to 5 and Comparative Examples 2 and 3 after storing the samples for one day, seven days, and 30 days after preparation at 4°C by irradiating the entire central area of the surface of samples with a laser beam at a wavelength of 520 nm and measuring the reflectance. The higher the reflectance (i.e. the smaller the change in luminance), the better the stability. The case in which the reflectance decreased 10% or more from the initial reflectance (reflectance immediately after preparation) in all samples was rated as "Bad" and the case in which all the samples exhibited a reflectance decrease of less than 10% was rated as "Good". The case in which some samples exhibited 10% or more of reflectance decrease and some samples exhibited less than 10% of reflectance decrease was rated as "Fair". In Example 5, the first and second reagents exhibited unevenness in the concentration in the central area and the neighborhood of the reagent spots. However, the unevenness presented no problem in practice. An analyte sample was dropped onto each of the above analytical test piece samples to measure the reflectance in the same manner as above to confirm the sensitivity. The sensitivity was rated as "Good" only in the case in which all samples after dropping the analyte samples exhibited a reflectance decrease of 20% or more from the reflectance before dropping the analyte samples. In addition, the samples were observed by the naked eye for inspection of unevenness in spots and unevenness in a 5 mm × 5 mm square.

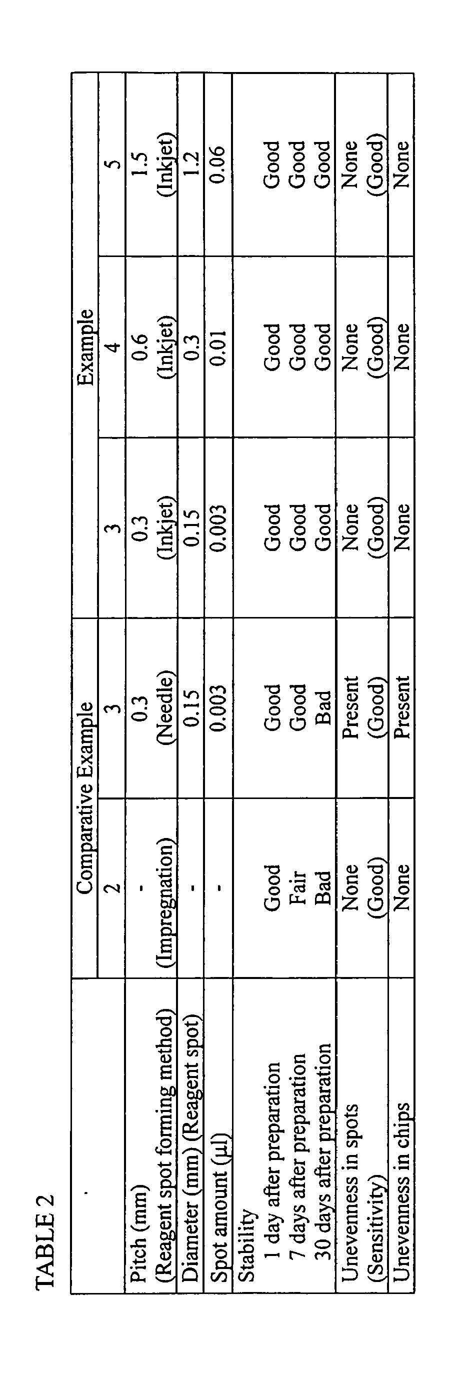

As shown in Table 2, it was confirmed that the reagent spots formed by aligning specific solutions (the first and second solutions in Examples 3 to 5) on the surface and inside of the support in a prescribed spot alignment pattern (a spot pitch) can be brought into contact and react with an analyte-containing sample introduced onto the surface of the support, and produce a detectable signals. It was further confirmed that an analytical test piece excelling in long term stability can be obtained. It was also confirmed that the inkjet method is more preferale than the needle method in preventing unevenness in the chip due to variation of the spot amount. Although two reagent solutions were used in Examples 3 to 5, taking stability into consideration, three or four types of reagent solutions may be used for forming the reagent spots. The experiment of Comparative Example 2 in Table 2 was the same as that of Comparative Example 1 in Table 1. Different results are believed to have been caused by fluctuation in the evaluation methods.

(Examples 6 and 7)
Fourth and fifth analyte samples for Examples 6 and 7, respectively, were prepared using alkaline phophataze instead of cow's milk (lactate dehydrogenase). Analytical test piece samples were prepared by using the inkjet method for forming reagent spots according to the following procedure and subjected to evaluation of long term stability and the other properties. As the first reagent, a mixture of 18.94 mM disodium p-nitrophenyl phosphate and 0.505 mM magnesium chloride (hexahydrate) (a first solution) was used and as the second reagent, a mixture of 5.05 mM 2-ethylaminoethanol and 0.505 mM magnesium chloride (hexahydrate) (a second solution) was used. Alkaline phophataze was used as an analyte sample. As supports, 5 mm × 5 mm boards with a thickness of 0.16 mm made of hydrophilic cellulose mixture ester with pore size of 0.8 µm were used. First and second reagent spots were prepared using the first and second solutions, the supports, and the injecting unit described in Japanese Patent Application Laid-open No. 2003-75305. In this instance, the first and second solutions in approximate amounts of 0.01 µl and 0.06 µl were respectively dispensed to each of the first and second reagent spots to form the first and second reagent spots with approximate pitches of 0.6 mm and 1.5 mm, and approximate diameters of 0.3 mm and 1.2 mm. Properties of the resulting fourth and fifth samples (Examples 6 and 7) were evaluated. The results are shown in Table 3.

(Comparative Example 4)
A third comparative analytical test piece sample (Comparative Example 4) was prepared using the liquid impregnation method as the method for forming reagent spots. Properties of the resulting sample were evaluated. The results are shown in Table 3. Specifically, the same support as used in Example 6 was impregnated with the same first and second reagents as used in Example 6 (a mixture of 1.01 mM 2-ethylaminoethanol, 75.75 mM disodium p-nitrophenyl phosphate, and 0.505 mM magnesium chloride (hexahydrate) as first and second solutions) in the same manner as in Comparative Example 2 to obtain a third comparative sample (Comparative Example 4). Properties of the sample were evaluated. The results are shown in Table 3.

Properties were evaluated in Examples 6 and 7 and Comparative Example 4 after storing the samples for one day, 100 days, and 150 days after preparation at 4°C by irradiating the entire central area of the surface of samples with a laser beam at a wavelength of 405 nm and measuring the absorbance, thereby determining the amount of p-nitrophenol which is the decomposition product of p-nitrophenyl phosphoric acid. Assuming that the absorbance at 405 nm at the start of storage is 100%, the closer the relative value to 100, the better the stability of the sample. The case in which the relative value decreased 10% or more from the initial value (absorbance immediately after preparation) in all samples was rated as "Bad" and the case in which all the samples exhibited a relative value decrease of less than 10% was rated as "Good". In Example 7, the first and second reagents exhibited unevenness in the concentration in the central area and the neighborhood of the reagent spots. However, the unevenness presented no problem in practice. An analyte sample was dropped onto each of the above analytical test piece samples to measure the reflectance in the same manner as above to confirm the sensitivity. The sensitivity was rated as "Good" only in the case in which all samples after dropping the analyte samples exhibited a reflectance decrease of 20% or more from the reflectance before dropping the analyte samples. In addition, the samples were observed by the naked eye for inspection of unevenness in spots and unevenness in a 5 mm × 5 mm square.

**TABLE 3**

| | | Comparative Example 4 | Example 6 | Example 7 |
|---|---|---|---|---|
| Pitch (mm) | | - | 0.6 | 1.5 |
| (Reagent spot forming method) | | (Impregnation) | (Inkjet) | (Inkjet) |
| Diameter (mm) (Reagent spot) | | - | 0.3 | 1.2 |
| Spot amount (µl) | | - | 0.01 | 0.06 |
| Stability | | | | |
| | I day after preparation | Good | Good | Good |
| | 100 days after preparation | Good | Good | Good |
| | 150 days after preparation | Bad | Good | Good |
| Unevenness in spots (Sensitivity) | | None (Good) | None (Good) | None (Good) |
| Unevenness in chips | | None | None | None |

As shown in Table 3, it was confirmed that the reagent spots formed by aligning specific solutions (the first and second solutions in Examples 6 and 7) on the surface and inside of the support in a prescribed spot alignment pattern (a spot pitch) can be brought into contact and react with an analyte-containing sample introduced onto the surface of the support, and produce a detectable signal. It was further confirmed that an analytical test piece excelling in long term stability with various types of solutions can be obtained. Although two reagent solutions were used in Examples 6 and 7, taking stability into consideration, three or four types of reagent solutions may be used for forming the reagent spots.

### INDUSTRIAL APPLICABILITY

The analytical test piece and the method for preparing the same of the present invention are effectively used for inspecting and analyzing samples containing an analyte (e.g. a body fluid, particularly urine, blood, etc. of humans and animals) and for preparing inspection chips and the like for analysis in various fields such as research, drug development, diagnosis, medical treatment, and the like.

## Claims

1. An analytical test piece comprising a support and reagent spots aligned on the surface of the support or on the surface and the inside of the support in a specified spot pattern (a spot pitch), wherein an analyte-containing sample introduced onto the surface of the support comes in contact with and reacts with the reagent spots aligned on the surface of the support or on the surface and inside of the support, thereby producing a detectable substance (a signal substance) or exhibiting a detectable property (a signal property),
the reagent spots being two or more types of reagent spots formed of any of two or more types of solutions which exhibit a given function when mixed with the other types of solutions, and
the sample introduced to the surface of the support coming in contact with the two or more types of reagent spots, causing the reagent spots to mix together, and reacting with the mixed reagent spots, thereby forming the signal substance or exhibiting the signal property.

2. The analytical test piece according to claim 1, wherein the support is a porous body.

3. The analytical test piece according to either claim 1 or 2, wherein the analyte is a human body fluid or animal body fluid.

4. The analytical test piece according to any one of claims 1 to 3, wherein at least one of the solutions forming the reagent spots comprises a coloring substance.

5. The analytical test piece according to any one of claims 1 to 4, wherein at least one of the solutions forming the reagent spots comprises a fluorescent substance.

6. The analytical test piece according to any one of claims 1 to 5, wherein at least one of the solutions forming the reagent spots comprises a water-soluble polymer.

7. The analytical test piece according to any one of claims 1 to 6, wherein water-soluble shields are disposed in spaces in which reagent spots are not aligned (between reagent spots) on the surface of the support or on the surface and inside of the support.

8. The analytical test piece according to any one of claims 1 to 7, wherein the reagent spots aligned in at least one area on the surface of the support or on the surface and inside of the support are types different from the reagent spots aligned in the other areas.

9. The analytical test piece according to any one of claims 1 to 8, wherein the concentration of reagent spots aligned in at least one area on the surface of the support or on the surface and inside of the support is different from the concentration of the reagent spots aligned in the other areas.

10. The analytical test piece according to any one of claims 1 to 9, wherein when the reagent spots are aligned not only on the surface, but also inside the support, the reagent spots are aligned so that those on the surface of the support exhibit the highest reactivity with the samples.

11. The analytical test piece according to any one of claims 1 to 9, wherein when the reagent spots are aligned not only on the surface, but also inside the support, the reagent spots are aligned so that those on the surface of the support have the highest concentration.

12. The analytical test piece according to any one of claims 1 to 11, wherein a surfactant is disposed in spaces in which reagent spots are not aligned (between reagent spots) on the surface of the support or on the surface and inside of the support.

13. The analytical test piece according to any one of claims 1 to 12, wherein a foaming agent is disposed in spaces in which reagent spots are not aligned (between reagent spots) on the surface of the support or on the surface and inside of the support.

14. The analytical test piece according to any one of claims 1 to 13, further provided with a supporting body for supporting the above support on the opposite surface (back) to the surface of the above support.

15. The analytical test piece according to any one of claims 1 to 14, wherein the cross-section of the reagent spots cut along a specified plane parallel to the surface of the support is oval or elliptical, or has the shape of a race track.

16. The analytical test piece according to any one of claims 1 to 15, wherein the distance between spot centers (spot pitch) of the same reagent (L2) is greater than the distance between spot centers (spot pitch) of the two different reagents (L1).

17. The analytical test piece according to any one of claims 1 to 16, wherein the diameter of the reagent spots is 0.5 mm or less and the distance (spot pitch) between the center of the different reagent spots (L1) is 0.6 mm or less.

18. The analytical test piece according to any one of claims 1 to 17, wherein an alignment ratio (A) of the reagent spots on the surface of the support or on the surface and inside of the support, expressed by the formula (A) = (S1/S2), satisfies an inequality of 0.01 < (A) < 0.814, wherein S 1 indicates the total cross-sectional areas of the reagent spots in a specified plane parallel to the surface of the support and S2 is the area of the surface of the support.

19. The analytical test piece according to any one of claims 1 to 18, wherein a spot pitch, which is the aligning pattern of the reagent spots on the surface of the support or on the surface and inside of the support, in a certain area differs from the spot pitch in other areas.

20. The analytical test piece according to any one of claims 1 to 19, further provided with confirmation reagent spots outside the area in which the reagent spots are aligned according to the specified pattern (spot pitch), for previously confirming proper alignment of the spots on the surface of the support or on the surface and in the inside of the support.

21. The analytical test piece according to any one of claims 1 to 20, wherein the amount of the solution (spot amount) in one reagent spot in at least one area on the surface of the support or on the surface and inside of the support differs from the spot amount in other areas.

22. A method for preparing an analytical test piece comprising reagent spots formed by aligning prescribed solutions on the surface of a support or on the surface and inside of the support in a prescribed spot alignment pattern (a spot pitch), with which an analyte-containing sample introduced onto the surface of the support is brought into contact and reacts, thereby producing a detectable substance (a signal substance) or exhibiting a detectable property (a signal property),
the method comprising providing two or more types of solutions, which are being mixed together when brought into contact with the above sample to be introduced and each of which exhibits a given function when mixed together, as the above prescribed solutions, and injecting the solutions onto the surface of the support or the surface and the inside of the support using an inkjet method to form two or more reagent spots, each consisting of any of the two or more types of solutions, so that the above sample introduced onto the surface of the support comes in contact with the reagent spots formed on the surface of the support or on the surface and inside of the support, causes the reagent spots to mix, and reacts with the mixed reagent spots to produce a signal substance or exhibit a signal property.

23. The method according to claim 22, wherein a liquid drop injector comprising a fluid channel substrate in which a fluid channel is formed, an actuator installed in the fluid channel substrate having a function of changing the volume of a cavity as a pressurizing chamber, a nozzle substrate attached to the bottom of the fluid channel substrate with nozzles formed therein, and a liquid receiver installed on the rear top of the fluid channel substrate is used in the inkjet method.

24. The method according to either claim 22 or 23, wherein a porous material is used as the support.

25. The method according to any one of claims 22 to 24, wherein the analyte is a human body fluid or animal body fluid.

26. The method according to any one of claims 22 to 25, wherein at least one of the solutions forming the reagent spots comprises a coloring substance.

27. The method according to any one of claims 22 to 26, wherein at least one of the solutions forming the reagent spots comprises a fluorescent substance.

28. The method according to any one of claims 22 to 27, wherein at least one of the solutions forming the reagent spots comprises a water-soluble polymer.

29. The method according to any one of claims 22 to 28, wherein water-soluble shields are disposed in spaces in which reagent spots are not aligned (between reagent spots) on the surface of the support or on the surface and inside of the support.

30. The method according to any one of claims 22 to 29, wherein the cross-section of the reagent spots cut along a specified plane parallel to the surface of the support is oval or elliptical, or has the shape of a race track.

31. The method according to any one of claims 22 to 30, wherein the reagent spots are aligned so that the distance between spot centers (spot pitch) of the same reagent (L2) may be greater than the distance between spot centers (spot pitch) of the two different reagents (L1).

32. The method according to any one of claims 22 to 31, wherein the diameter of the reagent spots is 0.5 mm or less and the distance (spot pitch) between the center of the different reagent spots (L1) is 0.6 mm or less.

33. The method according to any one of claims 22 to 32, wherein the reagent spots are aligned so that an alignment ratio (A) on the surface of the support or on the surface and inside of the support, expressed by the formula (A) = (S1/S2), satisfies an inequality of 0.01 < (A) < 0.814, wherein S1 indicates the total cross-sectional areas of the reagent spots in a specified plane parallel to the surface of the support and S2 is the area of the surface of the support.

34. The method according to any one of claims 22 to 33, wherein the reagent spots are aligned so that the spot pitch, which is the aligning pattern of the reagent spots on the surface of the support or on the surface and inside of the support, in a certain area may differ from the spot pitch in other areas.

35. The method according to any one of claims 22 to 34, wherein confirmation reagent spots are further provided outside the area in which the reagent spots are aligned according to the specified pattern (spot pitch), for previously confirming proper alignment of the spots on the surface of the support or on the surface and in the inside of the support.

36. The method according to claim 35, wherein each of the confirmation reagent spots contains one of the above-mentioned solutions.

37. The method according to any one of claims 22 to 36, wherein the reagent spots are aligned so that the reagent spots aligned in at least one area on the surface of the support or on the surface and inside of the support are types different from the reagent spots aligned in the other areas.

38. The method according to any one of claims 22 to 37, wherein the reagent spots are aligned so that the reagent spots aligned in at least one area on the surface of the support or on the surface and inside of the support have a concentration different from the reagent spots aligned in the other areas.

39. The method according to any one of claims 22 to 38, wherein when the reagent spots are aligned not only on the surface, but also inside the support, the reagent spots are aligned so that those on the surface of the support exhibit the highest reactivity with the samples.

40. The method according to any one of claims 22 to 39, wherein when the reagent spots are aligned not only on the surface, but also inside the support, the reagent spots are aligned so that those on the surface of the support exhibit the highest concentration.

41. The method according to any one of claims 22 to 40, wherein a surfactant is disposed in spaces in which reagent spots are not aligned (between reagent spots) on the surface of the support or on the surface and inside of the support.

42. The method according to any one of claims 22 to 41, wherein a foaming agent is disposed in spaces in which reagent spots are not aligned (between reagent spots) on the surface of the support or on the surface and inside of the support.

43. The analytical test piece according to any one of claims 22 to 42, wherein a supporting body for supporting the above support is provided on the opposite surface (back) to the surface of the above support.

44. The method according to any one of claims 22 to 43, wherein the reagent spots are formed by injecting liquid drops of the above two or more solutions using the inkjet method so that some solutions may be injected in a direction other than the vertical direction to the surface of the support.

45. The method according to any one of claims 22 to 44, wherein many very small independent droplets of the above solutions are produced.

46. The method according to any one of claims 22 to 45, wherein the amount of liquid drops forming one reagent spot (spot amount) is 0.1 µl or less.

47. The method according to any one of claims 22 to 46, wherein the amount of the solution (spot amount) in one reagent spot in at least one area on the surface of the support or on the surface and inside of the support differs from the spot amount in other areas.

48. The method according to any one of claims 22 to 47, wherein the reagent spots are formed by injecting droplets of the solution two or more times per one reagent spot.

49. The method according to any one of claims 22 to 48, wherein the reagent spots are produced by injecting the liquid drops of the above solutions while maintaining the temperature of the support at 40°C or less.

50. The method according to any one of claims 43 to 49, wherein the reagent spots are produced by injecting the liquid drops of the above solutions while the back of the support is separated from the supporting body.
